(19) Europäisches Patentamt
European Patent Office
Office européen des brevets

(11) Veröffentlichungsnummer : **0 133 935**
**B1**

(12) # EUROPÄISCHE PATENTSCHRIFT

(45) Veröffentlichungstag der Patentschrift : **23.12.87**

(21) Anmeldenummer : **84108419.7**

(22) Anmeldetag : **17.07.84**

(51) Int. Cl.⁴ : **C 07 C 65/24**, C 07 C 65/40, C 07 C 69/94, C 07 C 51/09, C 07 C 51/41, C 07 C 51/04, A 61 K 31/19, C 07 D303/04, C 07 C101/34, C 07 C 69/616

(54) **p-Oxibenzoesäurederivate, Verfahren zu ihrer Herstellung und ihre Verwendung zur Herstellung von Arzneimitteln mit hypolipämischer Wirkung.**

(30) Priorität : **20.07.83 DE 3326164**

(43) Veröffentlichungstag der Anmeldung : **13.03.85 Patentblatt 85/11**

(45) Bekanntmachung des Hinweises auf die Patenterteilung : **23.12.87. Patentblatt 87/52**

(84) Benannte Vertragsstaaten : **AT BE CH DE FR GB IT LI LU NL SE**

(56) Entgegenhaltungen :
**EP-A- 0 056 172**
**DE-A- 2 308 553**
**Die Akte enthält technische Angaben, die nach dem Eingang der Anmeldung eingereicht wurden und die nicht in dieser Patentschrift enthalten sind.**

(73) Patentinhaber : **Klinge Pharma GmbH**
**Berg-am-Laim-Strasse 129**
**D-8000 München 80 (DE)**

(72) Erfinder : **Grill, Helmut**
**Zugspitzstrasse 148**
**D-8011 Vaterstetten (DE)**
Erfinder : **Reiter, Friedemann**
**Zugspitzstrasse 36**
**D-8011 Putzbrunn (DE)**
Erfinder : **Löser, Roland**
**Fichtenweg 2**
**D-8133 Feldafing (DE)**
Erfinder : **Schliack, Michael**
**Josephsburgstrasse 4**
**D-8000 München 80 (DE)**
Erfinder : **Seibel, Klaus**
**Haberlstrasse 9**
**D-8032 Gräfelfing (DE)**

(74) Vertreter : **Wuesthoff, Franz, Dr.-Ing. et al**
**Patentanwälte Wuesthoff -v. Pechmann-Behrens-Goetz Schweigerstrasse 2**
**D-8000 München 90 (DE)**

**EP 0 133 935 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Zur medikamentösen Behandlung der Hyperlipidämie werden meist Aryloxyisoalkansäuren eingesetzt, von denen der bekannteste Wirkstoff der 2-(4'-Chlorphenoxy)-2-methylpropionsäure-ethyl-ester (CLOFIBRAT) ist. Es hat sich jedoch gezeigt, daß CLOFIBRAT einen nicht befriedigenden therapeutischen Effekt aufweist.

Bekannt ist auch, daß bestimmte, in Paraposition veretherte Benzoesäuren hypolipämische Eigenschaften besitzen, nämlich Benzoesäurederivate, die sich von Ethern des Glycerins (DE-PS 24 60 689) und des 1,3 Dihydroxy-acetons (DE-OS 27 35 856) ableiten.

Überraschenderweise wurde nun gefunden, daß p-Oxibenzoesäurederivate der allgemeinen Formel

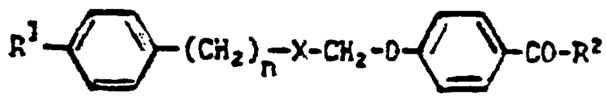

(1)

und ihre physiologisch verträglichen Salze, in der

$R^1$ = $—CH(CH_3)_2$ oder $—C(CH_3)_3$

n = 1 oder 2,

X =

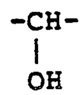 oder 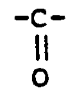 und

$R^2$ = —OH oder $—NHCH_2COOH$

bedeuten, ausgezeichnete hypolipämische Wirkung besitzen. Da die vorbekannten hypolipämisch wirkenden Substanzen Etherderivate des Glycerins bzw. Etherverbindungen des Dioxiacetons, einem Oxydationsprodukt des Glycerins sind, war zu schließen, das diese Substanzen aufgrund ihrer Glycerinstruktur, d. h. als Verwandte der natürlichen Glyceride wirken. Es war daher nicht vorhersehbar, daß die Etherverbindungen der p-Oxibenzoesäure oder deren in bestimmter Weise substituierten Säureamide mit zweiwertigen Alkoholderivaten, also Abkömmlinge des Glykols bzw. seines Oxydationsproduktes, ebenfalls eine hypolipämische Wirkung besitzen, zumal aus der EP-A-56 172 bekannt war, daß Phenoxy- und Thiophenoxyderivate ähnlicher Konstitution Wirkstoffe gegen Asthma und Bronchitis sein sollen.

Die Herstellung der erfindungsgemäßen Verbindungen kann in an sich bekannter Weise durchgeführt werden. Die für diese Synthesen notwendigen Ausgangsprodukte lassen sich beispielsweise nach folgenden Verfahren herstellen :

Durch Umsetzung von Epichlorhydrin mit Grignardverbindungen der allgemeinen Formel (5)

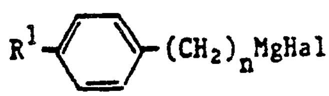

(5)

in der $R^1$ die in Anspruch 1 angegebene Bedeutung besitzt, n 0 oder 1 sein kann und Hal ein geeignetes Halogenatom darstellt, werden Verbindungen der allgemeinen Formel (6) erhalten,

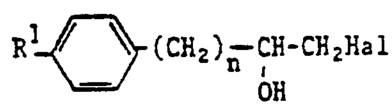

in der n 1 oder 2 ist, und die in Gegenwart von Alkalialkoholat in Epoxide der allgemeinen Formel (7) übergehen.

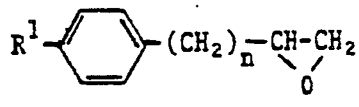

(7)

Verbindungen der allgemeinen Formel (7) reagieren mit 4-Hydroxybenzoesäureestern, z. B. mit 4-Hydroxybenzoesäuremethlyester, in Gegenwart von Alkali zu Verbindungen der allgemeinen Formel (8)

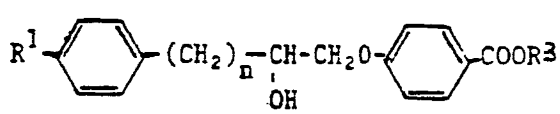

(8)

wobei $R^1$ und n die in Formel (1) angegebene Bedeutung besitzen und $R^3$ ein geeigneter Alkylrest ist. Durch alkalische Verseifung und anschließendem Ansäuern mit Mineralsäure werden daraus die erfindungsgemäßen Verbindung der allgemeinen Formel (9) erhalten.

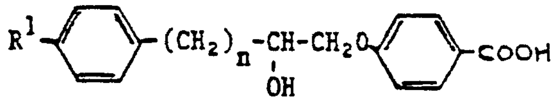
(9)

Eine Umsetzung mit Acetylchlorid liefert Verbindungen der allgemeinen Formel (10)

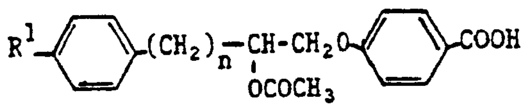
(10)

die sich mit Thionylchlorid in Säurechloride der allgemeinen Formel (11) überführen lassen.

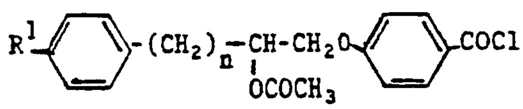
(11)

Aus den Säurechloriden der allgemeinen Formel (11) werden nach Umsetzung mit Natriumglycinat und anschließender alkalischer Verseifung, durch Ansäuern mit Mineralsäure die erfindungsgemäßen Verbindungen der allgemeinen Formel (12) in Freiheit gesetzt,

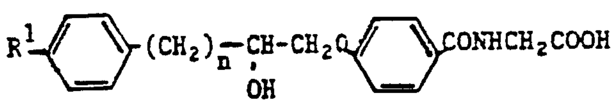
(12)

wobei $R^1$ und n die in Formel (1) angegebene Bedeutung besitzen. Durch selektive Oxidation mit Dimethylsulfoxid werden Verbindungen der allgemeinen Formeln (9) und (12) in Ketone der allgemeinen Formel (13) übergeführt,

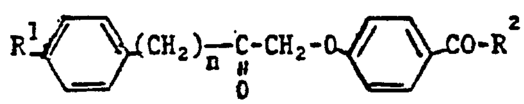
(13)

in der $R^1$, n und $R^2$ die in Formel (1) angegebene Bedeutung besitzen.

Die Überlegenheit der beanspruchten Verbindungen gegenüber dem bereits seit langem in die Therapie eingeführten CLOFIBRAT läßt sich anhand der lipidsenkenden Wirkung eindeutig belegen.

Die lipidsenkende Wirkung wurde an Gruppen von jeweils 10 normal gefütterten, normolipämischen, männlichen 200 bis 220 g schweren Wistar-Ratten geprüft.

Die Testverbindungen wurden in einer wäßrigen Lösung von 0,25 % Agar und 0,85 % Natriumchlorid aufgenommen und oral verabreicht. Nach Applikation von 4 × 100 mg/kg über einen Zeitraum von drei Tagen wurden die Tiere im Anschluß an einen vierstündigen Futterentzug durch Herzpunktion getötet.

Die Bestimmung des Gesamtcholesterins (TC) wurde enzymatisch nach Siedel, J. et al [J. Clin. Chem. Clin. Biochem. 19, 838 (1981)] durchgeführt. Die quantitative Analyse der Triglyceride (TG) erfolgte enzymatisch mit im Handel befindlichen Test-Kits [Boehringer/Mannheim] am Autoanalyzer « Cobas/Bio » [Hoffmann-La Roche/Basel] nach Wahlefeld, A. W., [Bergmeyer, H. U., « Methoden der enzymatischen Analyse », 3. Auflage, Bd. II, Verlag Chemie, Weinheim 1974, Seite 1878].

Im folgenden wird die Erfindung anhand von Ausführungsbeispielen näher erläutert.

## Beispiel 1

4-[4-(4'-tert.Butylphenyl)-2-hydroxybutoxy] benzoesäure

a) 4-(4'-tert.Butylphenyl)-1,2-epoxybutan

Die aus 12,15 g (0,50 g-Atom) Magnesiumspänen und 91,7 g (0.50 Mol) 4-tert.Butylbenzylchlorid in 550 ml trockenem Ether frisch bereitete Grignardlösung wird unter Rühren in die vorgekühlte Lösung aus 92,5 g (1.00 Mol) Epichlorhydrin in 165 ml trockenem Ether getropft, so daß die Temperatur — 40 °C nicht übersteigt. Nach beendeter Zugabe entfernt man das Kühlbad, läßt einige Stunden bei Raumtemperatur stehen und hydrolysiert dann mit Eiswasser und verd. Salzsäure. Die Etherphase wird abgetrennt, mit

Wasser gewaschen und über Natriumsulfat getrocknet. Nach Abziehen des Ethers wird die Badtemperatur auf 80 °C erhöht und die flüchtigen Anteile bei 0.1 mbar sorgfältig entfernt. Der Rückstand wird in 270 ml Methanol aufgenommen und bei Raumtemperatur eine frisch bereitete Lösung von 6,90 g (0.30 g-Atom) Natrium in 200 ml Methanol hinzugefügt. Nach drei Stunden wird das abgeschiedene Natriumchlorid abgetrennt, das Filtrat i. Vak. eingeengt, der Rückstand in Ether aufgenommen und mit Wasser gewaschen. Nach dem Trocknen über Natriumsulfat wird das Lösungsmittel entfernt und der ölige Rückstand i. Vak. destilliert. Farblose Flüssigkeit vom Siedepunkt 79-81 °C/0.1 mbar.

Ausbeute 40,0 g (39 %).

$^1$H-NMR-Spektrum ($CDCl_3$) :

| | |
|---|---|
| 1.33 s (9) | $(CH_3)_3C$ |
| 1.53 bis 2.07 m (2) | $ArCH_2CH_2$ |
| 2.23 bis 3.13 m (5) | $ArCH_2CH_2$ 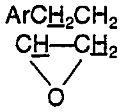 |
| 7.17 (Zentrum) m (4) | Aromat |

(NMR-Spektrum, aufgenommen bei 60 MHZ.

Die chemischen Verschiebungen sind in ppm gegen TMS (δ = 0.0) angegeben, die relativen Intensitäten sind in Klammern beigefügt. s = Singulett ; D = Dublett ; t = Triplett ; q = Quartett ; m = Multiplett).

b) 4-[4-(4'-tert.Butylphenyl)-2-hydroxybutoxy] benzoesäuremethylester

40,0 g (0.196 Mol) 4-(4'-tert.Butylphenyl)-1,2-epoxybutan werden mit 31,7 g (0.214 Mol) 4-Hydroxybenzoesäuremethylester und 1,38 g Kaliumhydroxid in 170 ml Methanol 60 Stunden unter Rückfluß erhitzt. Anschließend engt man i. Vak. ein, nimmt den Rückstand in Ether auf, schüttelt mit 1 N Natronlauge und Wasser aus. Nach dem Trocknen über Natriumsulfat wird der Ether i. Vak. entfernt und der ölige Rückstand aus Diisopropylether kristallisiert. Farblose Kristalle vom Schmelzpunkt 77-78 °C ; Ausbeute 43,3 g (62 %).

$^1$H-NMR-Spektrum ($CDCl_3$) :

| | |
|---|---|
| 1.33 s (9) | $(CH_3)_3C$ |
| 1.63 bis 2.23 m (2) | $ArCH_2CH_2$ |
| 2.47 d (1) | OH |
| 2.60 bis 3.07 m (2) | $ArCH_2CH_2$ |
| 3.77 bis 4.33 m und 3.90 s (6) | CH—$CH_2O$<br>$CH_3O$ |
| 6.73 bis 8.23 m (8) | Aromat |

c) Herstellung von 4-[4-(4'-tert.Butylphenyl)-2-hydroxybutoxy] benzoesäure

35,6 g (0.10 Mol) 4-[4-(4'-tert.Butylphenyl)-2-hydroxybutoxy] benzoesäuremethylester werden mit einer Lösung von 18,0 g (0.32 Mol) Kaliumhydroxid in 125 ml Ethanol 1,5 Stunden unter Rückfluß erhitzt. Anschließend wird das Lösungsmittel i. Vak. entfernt, der Rückstand in Wasser aufgenommen, mit Ether extrahiert und die wässrige Phase mit konz. Salzsäure angesäuert. Das sich abscheidende Produkt wird in Ethylacetat aufgenommen und die organische Phase mit Wasser gewaschen. Nach dem Trocknen über Natriumsulfat wird das Lösungsmittel i. Vak. entfernt und der Rückstand zweimal aus Acetonitril kristallisiert. Farblose Kristalle vom Schmelzpunkt 142-143 °C ; Ausbeute 23.6 g (69 %).

$C_{21}H_{26}O_4$ (342.4)

Mol.-Gew. : 342 (massenspektrometrisch mittels Elektronenstoß-Ionisation (70 eV) bestimmt)

IR-Spektrum (KBr) : ν(OH) 3 600 bis 2 500 $cm^{-1}$
ν(C=O) 1 680 $cm^{-1}$

$^1$H-NMR-Spektrum ($CDCl_3$) :

| | |
|---|---|
| 1.30 s (9) | $(CH_3)_3C$ |
| 1.73 bis 2.17 m (2) | $ArCH_2CH_2$ |
| 2.63 bis 3.03 m (2) | $ArCH_2CH_2$ |
| 3.80 bis 4.27 m (3) | $CHCH_2O$ |
| 6.67 bis 8.20 m (10) | Aromat<br>OH<br>COOH |

In analoger Weise zu dem Beispiel 1 wurden weitere Verbindungen der allgemeinen Formel (9) hergestellt und in nachfolgender Tabelle 1 mit den Schmelzpunkten aufgeführt.

Tabelle 1

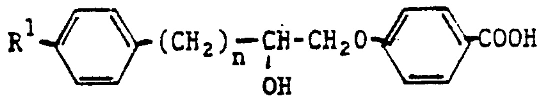

(9)

| Nr. | $R^1$ | n | Schmp. (°C)[x)] | Lösungsmittel[xx)] |
|---|---|---|---|---|
| 1 | $(CH_3)_2CH$ | 1 | 154 - 156 | (a) |
| 2 | $(CH_3)_3C$ | 1 | 189 - 190 | (b) |
| 3 | $(CH_3)_3C$ | 2 | 142 - 143 | (b) |

[x)] Die Schmelzpunkte wurden mit einem Kofler-Heiztisch-Mikroskopermittelt und sind nicht korrigiert.
[xx)] Kristalle aus
(a) : Ethylacetat/Acetonitril
(b) : Acetonitril

Beispiel 2

N-Carboxymethyl-4-[4-(4'-tert.Butylphenyl)-2-hydroxybutoxy] benzamid

a) 4-[4-(4'-tert.Butylphenyl)-2-acetoxybutoxy] benzoesäure

34,2 g (0.10 Mol) 4-[4-(4'-tert.Butylphenyl)-2-hydroxybutoxy]-benzoesäure und 0,30 g (2.2 mMol) wasserfreies Zinkchlorid werden in 240 ml wasserfreier Essigsäure gelöst und mit 15,8 ml (0.22 Mol) Acetylchlorid versetzt. Nach dreistündigem Rühren bei Raumtemperatur gießt man die Reaktionslösung in Eiswasser. Das abgeschiedene Produkt wird in Ether aufgenommen und mit Wasser gewaschen. Nach dem Trocknen über Natriumsulfat wird der Ether i. Vak. entfernt und das farblose, kristalline Rohprodukt ohne Reinigung zur weiteren Umsetzung verwendet. Rohausbeute 37,4 g (97 %).

$^1$H-NMR-Spektrum ($CDCl_3$)

| | |
|---|---|
| 1.30 s (9) | $(C\underline{H}_3)_3C$ |
| 1.83 bis 2.33 m | $ArCH_2C\underline{H}_2$ |
| und 2.07 s (5) | $C\underline{H}_3CO$ |
| 2.50 bis 2.93 m (2) | $ArC\underline{H}_2CH_2$ |
| 4.10 d (2) | $C\underline{H}_2O$ |
| 5.27 m (1) | $C\underline{H}O$ |
| 6.77 bis 8.27 m (8) | Aromat |
| 10.7 breites s (1) | $COO\underline{H}$ |

b) Herstellung von N-Carboxymethyl-4-[4-(4'-tert.Butylphenyl)-2-hydroxybutoxy] benzamid

37,4 g (0.097 Mol) rohe 4-[4-(4'-tert.Butylphenyl)-2-acetoxybutoxy] benzoesäure und 12 ml (0.165 Mol) Thionylchlorid in 240 ml Toluol werden 3,5 Stunden unter Rückfluß erhitzt. Anschließend destilliert man Lösungsmittel und überschüssiges Thionylchlorid i. Vak. ab und nimmt den öligen Rückstand in 250 ml trockenem Dioxan auf. Diese Lösung tropft man unter lebhaftem Rühren bei 5 bis 7 °C in eine Lösung von 29,5 g (0.39 Mol) Glycin und 15.6 g (0.39 Mol) Natriumhydroxid in 350 ml Wasser. Anschließend rührt man drei weitere Stunden bei Raumtemperatur, fügt dann 30 g Natriumhydroxid hinzu und erwärmt zwei Stunden auf 50 °C. Nach dem Abkühlen verdünnt man mit Wasser und säuert mit konz. Salzsäure an. Das ausgefällte Endprodukt wird in Ethylacetat aufgenommen und zweimal mit Wasser gewaschen. Nach dem Trocknen über Natriumsulfat wird das Lösungsmittel i. Vak. entfernt und der feste Rückstand aus Acetonitril kristallisiert. Farblose Kristalle vom Schmelzpunkt 112-114 °C ; Ausbeute 26,0 g (67 %).

$C_{23}H_{29}NO_5$ (399,5)

Mol.-Gew. : 399 (massenspektrometrisch bestimmt)

IR-Spektrum (KBr) : ν(O—H) 3 600 bis 2 400 $cm^{-1}$
ν(C=O) 1 720 $cm^{-1}$
ν(N—H) 3 400 $cm^{-1}$

$^1H$-NMR-Spektrum ($d_6$-Aceton/$d_6$-DMSO)

| | |
|---|---|
| 1.30 s (9) | $(C\underline{H}_3)_3C$ |
| 1.67 bis 2.20 m (2) | $ArCH_2C\underline{H}_2$ |
| 2.60 bis 3.03 m (2) | $ArC\underline{H}_2CH_2$ |
| 3.77 bis 4.23 m (5) | $C\underline{H}C\underline{H}_2$, $C\underline{H}_2N$ |
| 6.83 bis 8.23 m (10) | Aromat, N$\underline{H}$, COO$\underline{H}$ |

In analoger Weise zum Beispiel 2 wurden weitere Verbindungen der allgemeinen Formel (12) hergestellt und in nachfolgender Tabelle 2 mit Schmelzpunkten aufgeführt.

Tabelle 2

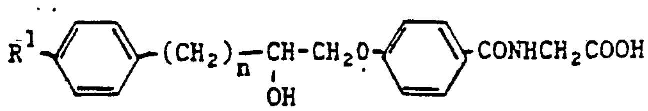

(12)

| Nr. | $R^1$ | n | Schmp. (°C) | Lösungsmittel x) |
|---|---|---|---|---|
| 4 | $(CH_3)_3C$ | 1 | 153 - 154 | (a) |
| 5 | $(CH_3)_2CH$ | 2 | 128 - 130 | (b) |
| 6 | $(CH_3)_3C$ | 2 | 112 - 114 | (a) |

x) Kristalle aus
(a) : Acetonitril
(b) : Ethylacetat

## Beispiel 3

4-[4-(4'-tert.Butylphenyl)-2-oxobutoxy] benzoesäure

Zu 14,5 ml (0.2 Mol) wasserfreiem Dimethylsulfoxid in 180 ml trockenem Dichlormethan werden unter starkem Rühren bei —70 °C 21 ml (0.15 Mol) Trifluoressigsäureanhydrid in 21 ml trockenem Dichlormethan getropft. Die dabei entstehende Suspension wird noch 10 Min. gerührt und anschließend langsam mit einer Lösung von 3.42 g (0.10 Mol) 4-[4-(4'-tert.Butylphenyl)-2-hydroxybutoxy] benzoesäure in 73 ml trockenem Dichlormethan und 18 ml Dimethylsulfoxid versetzt, so daß die Temperatur nicht über —50 °C ansteigt. Nach 30 Min. werden 42 ml Triethylamin vorsichtig zugefügt und das Reaktionsgemisch durch Entfernen des Kältebads allmählich auf Raumtemperatur gebracht. Die Reaktionslösung wird mehrmals mit Wasser gewaschen und nach dem Trocknen über Natriumsulfat das Lösungsmittel i. Vak. entfernt. Der Rückstand wird aus Isopropylacetat/Chlorbutan kristallisiert. Farblose Kristalle vom Schmelzpunkt 149-153 °C ;

Ausbeute 12,2 g (36 %).
$C_{21}H_{24}O_4$ (340.4)
Mol.-Gew. : 340 (massenspektrometrisch bestimmt)
IR-Spektrum (KBr) : ν(O—H) 3 600 bis 2 500 $cm^{-1}$
ν(C=O) 1 710 $cm^{-1}$ (Keton), 1 $675^{-1}$ (Carbonsäure)

$^1H$-NMR-Spektrum ($CDCl_3$)

| | |
|---|---|
| 1.30 s (9) | $(C\underline{H}_3)_3C$ |
| 2.93 s (4) | $C\underline{H}_2C\underline{H}_2$ |
| 4.57 s (2) | $C\underline{H}_2O$ |
| 6.70 bis 8.23 m (8) | Aromat |
| 9.60 breites s (1) | COO$\underline{H}$ |

In analoger Weise zu dem Beispiel 3 wurden weitere Verbindungen der allgemeinen Formel (13) hergestellt und in nachfolgender Tabelle 3 mit Schmelzpunkten aufgeführt.

Tabelle 3

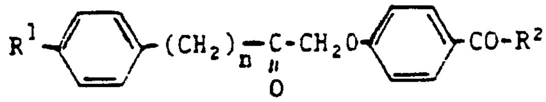

(13)

| Nr. | $R^1$ | n | $R^2$ | Schmp.(°C) | Lösungsmittel*) |
|---|---|---|---|---|---|
| 7 | $(CH_3)_3C$ | 1 | OH | 163 - 164 | (a) |
| 8 | $(CH_3)_3C$ | 2 | OH | 149 - 153 | (b) |
| 9 | $(CH_3)_3C$ | 1 | $NHCH_2COOH$ | 69 - 73 | (c) |
| 10 | $(CH_3)_2CH$ | 2 | $NHCH_2COOH$ | 133 - 134 | (d) |

* Kristalle aus
(a) : Acetonitril
(b) : Chlorbutan/Isopropylacetat
(c) : Chlorbutan
(d) : Ethylacetat

## Beispiel 4

4-[4-(4'-tert.Butylphenyl)-2-oxo-butoxy] benzoesäure enthaltendes Arzneimittel

250 g 4-[4-(4'-tert.Butylphenyl)-2-oxo-butoxy] benzoesäure werden mit 250 g Polyethylenglykol vermischt und nach dem Schererverfahren in eintausend Weichgelatinekapseln abgefüllt, die je 250 mg Wirkstoff enthalten.

In der nachfolgenden Tabelle sind Ergebnisse von pharmakologischen Versuchen angegeben, wobei die lipidsenkende Wirkung ausgedrückt ist als die prozentuale Senkung des Gesamtcholesterins und der Triglyceride gegenüber der Kontrolle. In der Spalte X ist der arithmetische Mittelwert und in der Spalte $S_x$ die Standardabweichung angegeben.

Tabelle 4

Prozentuale Senkung der Triglycerid (TG)- und Gesamtcholesterin (TC)-Spiegel im Rattenserum nach oraler Applikation der Testsubstanzen

| Verbindungsnummer | % Senkung | | | | | |
|---|---|---|---|---|---|---|
| | TG | | | TC | | |
| | $\bar{X}$ | ± | $S_x$ | $\bar{X}$ | ± | $S_x$ |
| Vergleichssubstanz Clofibrat | 29,2 | ± | 19,4 | 14,5 | ± | 12,0 |
| 1 | 53,3 | ± | 12,2 | 2,7 | ± | 9,2 |
| 2 | 40,6 | ± | 13,9 | 15,6 | ± | 14,4 |
| 3 | 52,1 | ± | 14,3 | 55,3 | ± | 16,5 |
| 5 | 57,5 | ± | 11,7 | 6,4 | ± | 14,6 |
| 6 | 69,0 | ± | 10,3 | 48,7 | ± | 9,2 |
| 7 | 50,6 | ± | 11,5 | 22,2 | ± | 7,4 |
| 8 | 48,9 | ± | 24,1 | 53,3 | ± | 15,3 |
| 9 | 52,1 | ± | 12,0 | 21,9 | ± | 15,4 |

Für die therapeutische Anwendung als hypolipämische Arzneimittel werden die neuen Verbindungen der allgemeinen Formel (1) und ihre Salze bevorzugt oral verabreicht. Gewöhnlich beträgt die orale Tagesdosis bei Erwachsenen 0,1 bis 5 g, vorzugsweise 0,3 bis 2 g.

Die Wirkstoffe können zur oralen Verabreichung in üblicher Weise galenisch verabreicht werden. Als pharmazeutische Trägerstoffe eignen sich gängige Hilfsstoffe wie Lactose, Saccharose, Mannit, Kartoffel- oder Maisstärke, Cellulosederivate oder Gelatine, gegebenenfalls unter Zusatz von Gleitmitteln, wie z. B. Magnesium- oder Calciumstearat, sowie Polyethylenglykole.

Bevorzugte Verabreichungsformen sind Steckkapseln aus Hartgelatine sowie geschlossene Weichgelatinekapseln. In Steckkapseln kann gegebenenfalls der reine Wirkstoff evtl. mit einem geringen Zusatz an Gleitmitteln enthalten sein. Bei entsprechenden physikalischen Eigenschaften des Wirkstoffes wird eine Verarbeitung zu Granulaten bevorzugt, wobei als Hilfsstoffe Kartoffel- oder Maisstärke, mikrokristalline Cellulose, Cellulosederivate, Gelatine oder auch hochdisperse Kieselsäuren mitverwendet werden.

Bei Konfektionierung in Weichgelatinekapseln wird der reine Wirkstoff in geeigneten Flüssigkeiten gelöst oder suspendiert, so z. B. in flüssigen Polyethylenglykolen oder Pflanzenölen.

**Patentansprüche**

1. p-Oxibenzoesäurederivate der allgemeinen Formel

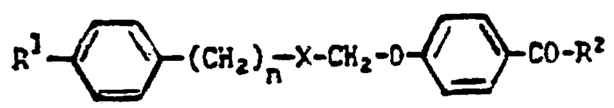

worin
$R^1$ = $-CH(CH_3)_2$, $-C(CH_3)_3$
n = 1, 2
X = $-CH(OH)-$, $-CO-$
$R^2$ = $-OH$, $-NHCH_2COOH$ bedeuten,
sowie ihre physiologisch verträglichen Salze.

2. 4-[4-(4'-tert.Butylphenyl)-2-hydroxybutoxy] benzoesäure.

3. Verfahren zur Herstellung der Benzoesäurederivate nach Anspruch 1, dadurch gekennzeichnet, daß man in an sich bekannter Weise

a) Verbindungen der allgemeinen Formel (2)

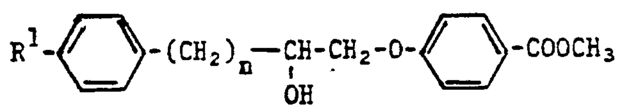

(2)

in der $R^1$ und n die in Anspruch 1 beschriebene Bedeutung besitzen mit Alkali verseift, durch Zugabe von Mineralsäure die Carboxylverbindung in Freiheit setzt und nach Kristallisation gegebenenfalls mit Basen zu den entsprechenden Salzen umsetzt ;

b) Verbindungen der allgemeinen Formel (3)

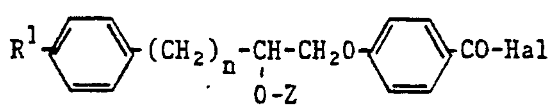

(3)

in der $R^1$ und n die in Anspruch 1 beschriebene Bedeutung besitzen, Z eine Schutzgruppe, z. B. Acetyl bedeutet und Hal ein Halogenatom, bevorzugt Chlor darstellt, mit Natriumglycinat in an sich bekannter Weise umsetzt, anschließend mit Alkali die Schutzgruppe abspaltet, durch Zugabe von Mineralsäure die Carboxylverbindung in Freiheit setzt und nach Kristallisation gegebenenfalls mit Basen zu den entsprechenden Salzen umsetzt ; oder

c) Verbindungen der allgemeinen Formel (4)

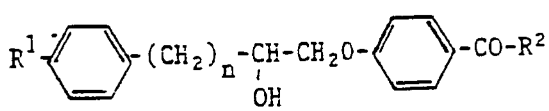

(4)

in der $R^1$, n und $R^2$ die in Anspruch 1 beschriebene Bedeutung besitzen, einer Oxidation mit Dimethylsulfoxid unterzieht, das Oxidationsprodukt kristallisiert und gegebenenfalls mit Basen zu den entsprechenden Salzen umsetzt.

4. Verwendung der p-Oxibenzoesäurederivate der allgemeinen Formel :

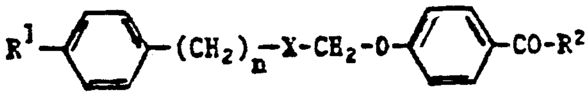 (1)

und ihre physiologisch verträglichen Salze, in der
$R^1$ = —H oder eine geradkettige und verzweigte $C_1$-$C_4$-Alkylgruppe,
n = 1 oder 2,
X =

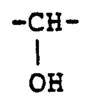 oder 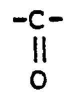 und

$R^2$ = —OH oder —$NHCH_2COOH$
bedeuten, zur Herstellung eines Arzneimittels mit hypolipämischer Wirkung.

**Claims**

1. P-oxybenzoic acid derivatives represented by the general formula

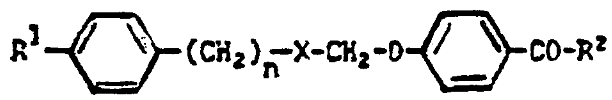

wherein
$R^1$ = —$CH(CH_3)_2$, —$C(CH_3)_3$
n = 1, 2
X = —CH(OH)—, —CO—
$R^2$ = —OH, —$NHCH_2COOH$
as well as the physiologically compatible salts thereof.

2. 4-[4'-tert.butylphenyl)-2-hydroxybutoxy] benzoic acid.

3. Method for the preparation of the benzoic acid derivatives according to claim 1 characterized in that, in a manner known per se,

a) compounds represented by the general formula (2)

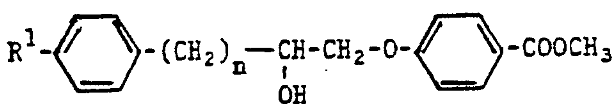 (2)

wherein $R^1$ and n have the meaning defined in claim 1, are saponified with alkali, the carboxyl compound being released by the addition of mineral acid and converted after crystallization optionally with bases to the corresponding salts ;

b) compounds represented by the general formula (3)

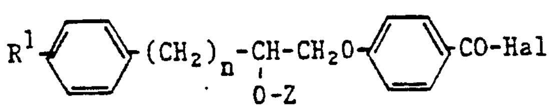 (3)

wherein $R^1$ and n have the meaning defined in claim 1, Z is a protective group, for example acetyl, and Hal represents a halogen atom, preferably chlorine, are reacted with sodium glycinate in a manner known per se, whereupon the protective group is cleaved off with an alkali, the carboxyl compound being released by the addition of mineral acid and after crystallization being converted optionally with bases to the corresponding salts ; or

c) compounds represented by the general formula (4)

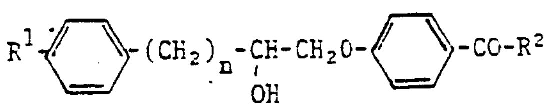 (4)

wherein $R^1$, n and $R^2$ have the meaning defined in claim 1, are oxidized with dimethylsulfoxide, the oxidation product being crystallized and optionally converted with bases to the corresponding salts.

4. The use of p-oxybenzoic acid derivatices represented by the general formula :

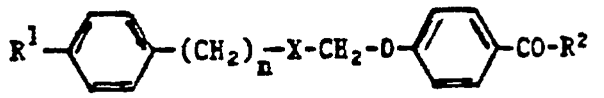 (1)

and of the physiologically compatible salts thereof, wherein

$R^1$ = —H or a straight-chain and branched $C_1$-$C_4$-alkyl group

n = 1 or 2

X =

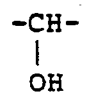 or 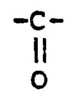 and

$R^2$ = —OH or —$NHCH_2COOH$

for the preparation of a medicament having hypolipemic activity.

**Revendications**

1. Dérivés d'acide p-oxybenzoïque répondant à la formule générale (1) :

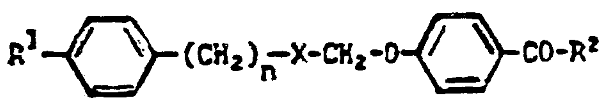 (1)

dans laquelle

$R^1$ = —$CH(CH_3)_2$, —$C(CH_3)_3$

n = 1, 2

X = —CH(OH)—, —CO—

$R^2$ = —OH, —$NHCH_2COOH$

ainsi que leurs sels physiologiquement acceptables.

2. L'acide 4-[4-(4'-tert.butylphényl)-2-hydroxybutoxy] benzoïque.

3. Procédé pour la préparation des dérivés d'acide benzoïque selon la revendication 1, caractérisé en ce que, de manière en soi connue :

a) on saponifie à l'aide d'un alcali un composé répondant à la formule générale (II) :

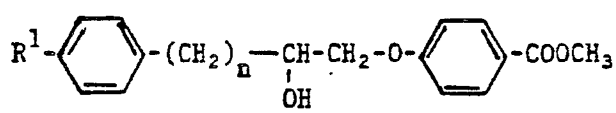 (2)

dans laquelle $R^1$ et n ont les significations données à la revendication 1, on libère le composé carboxylique par addition d'un acide minéral, et, le cas échéant, on le transforme en un sel correspondant par cristallisation avec une base ;

b) on fait réagir, de manière en soiconnue, du glycinate de sodium avec un composé de formule générale (III) :

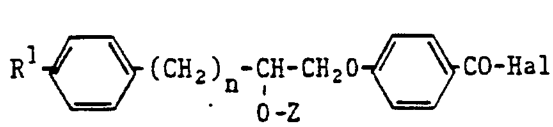 (3)

dans laquelle $R^1$ et n ont les significations données à la revendication 1, Z représente un groupe protecteur par exemple acétyle, et Hal représente un atome d'halogène de préférence du chlore, on sépare ensuite le groupe protecteur à l'aide d'un alcali, on libère le composé carboxylique par addition d'un acide minéral et, le cas échéant, on le transforme en un sel correspondant par cristallisation avec une base ;

c) on soumet à une oxydation, à l'aide de diméthylsulfoxide, un composé de formule générale (IV) :

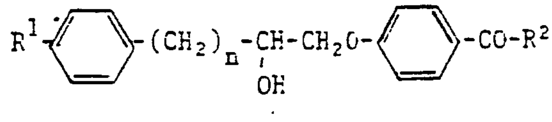 (4)

dans laquelle $R^1$, n et $R^2$ ont les significations données à la revendication 1, on cristallise le produit d'oxydation et, le cas échéant, on le transforme en un sel correspondant par cristallisation avec une base.

4. Utilisation des dérivés d'acide p-oxibenzoïque de formule générale (1) :

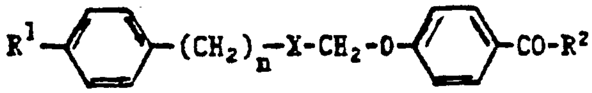 (1)

ainsi que de leurs sels physiologiquement acceptables, dans laquelle

$R^1$ = —H ou un groupe alcoyle linéaire ou ramifié en $C_1$ à $C_4$

n = 1 ou 2

X =

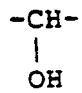 ou 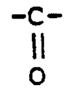 et

$R^2$ = —OH ou —$NHCH_2COOH$

pour la fabrication de médicaments présentant une activité hypolipémique.